# EUROPÄISCHE PATENTSCHRIFT

(11) **EP 2 148 710 B1**
(45) Veröffentlichungstag und Bekanntmachung des Hinweises auf die Patenterteilung: **02.10.2019**
(21) Anmeldenummer: 08748875.5
(22) Anmeldetag: 09.04.2008
(51) Int. Cl.: A61M 5/315, A61M 5/24

(54) **INJEKTIONSVORRICHTUNG ZUR ABGABE EINES MEDIKAMENTS**
INJECTION DEVICE FOR DELIVERING A MEDICATION
DISPOSITIF D'INJECTION POUR ADMINISTRER UN MÉDICAMENT

(30) Priorität: 18.04.2007 DE 102007018696
(43) Veröffentlichungstag der Anmeldung: 03.02.2010
(73) Patentinhaber: Sanofi-Aventis Deutschland GmbH, 65929 Frankfurt am Main (DE)
(72) Erfinder: KIETZMANN, Hardy, 65926 Frankfurt am Main (DE)
(74) Vertreter: Keil & Schaafhausen Patent- und Rechtsanwälte PartGmbB
(86) Internationale Anmeldenummer: PCT/EP2008/002787
(87) Internationale Veröffentlichungsnummer: WO 2008/128645

(56) Entgegenhaltungen:
- EP-A- 0 688 571
- EP-A- 1 557 189
- WO-A-99/64092
- WO-A-02/064199
- WO-A-2006/037434
- US-A1- 2005 197 626

## Beschreibung

Die Erfindung betrifft eine Injektionsvorrichtung zur Abgabe eines Medikaments.

Das Dokument EP 1 557 189 A offenbart eine Injektionsvorrichtung zur Abgabe eines Medikaments mit den Merkmalen des Oberbegriffs des Anspruchs 1.

Aufgabe der Erfindung ist, eine Injektionsvorrichtung bereitzustellen, mit der eine verbesserte Einstellung der abzugebenden Injektionsdosis erreicht wird.

Eine weitere Aufgabe der Erfindung ist, eine medizinische Injektionsvorrichtung bereitzustellen, mit der die Dosiergenauigkeit für den Anwender verbessert werden kann.

Diese Aufgabe wird mit den Merkmalen des unabhängigen Anspruchs 1
gelöst. Weitere Ausführungsformen sind in den auf diesen rückbezogenen Unteransprüchen angegeben.

Durch die erfindungsgemäße Lösung wird eine mechanische Verfestigung des Abgabemechanismus und/oder des Dosis-Einstellmechanismus und somit der Einstellmechanik bei verbesserter Ablesbarkeit der eingestellten Dosis erreicht.

Durch eine Ausführungsform der erfindungsgemäßen Lösung kann eine vorteilhafte Abdichtung des Inneren des Gehäuses der Injektionsvorrichtung gegen Verschmutzungen erreicht werden.

Erfindungsgemäß kann weiterhin die Einsteck-Hülse in das distale Gehäuseteil durch eine, durch entsprechende Abmessungen des erhabenen Abschnitts und der Ausnehmung bewirkte Passverbindung zwischen erhabenem Abschnitt und der Ausnehmung eingesetzt werden, so dass die axiale Position der Anzeige-Hülse genauer einstellbar ist.

Dadurch können gegebenenfalls Fertigungsungenauigkeiten an Bestandteilen der Injektionsvorrichtung ausgeglichen werden.

Gegenstand der Erfindung ist daher eine Injektionsvorrichtung zur Abgabe eines Medikaments gemäß Anspruch 1, aufweisend:
- ein Kartuschen-Modul zur Aufnahme einer Kartusche enthaltend ein Injektionsmittel und gegebenenfalls mit einer Aufnahme zum Anbringen einer Nadel, die an einem Ende der Kartusche angebracht ist und durch die das Injektionsmittel injiziert werden kann,
- einen Abgabemechanismus, durch dessen Betätigung die Abgabe des Injektionsmittels erreicht wird,
- einen Dosis-Einstellmechanismus zur Einstellung der Dosis, mit der das Injektionsmittel bei deren Betätigung des Abgabemechanismus abgegeben wird, wobei der Dosis-Einstellmechanismus aufweist: ein Gehäuseteil, in das zumindest ein Teil der Abgabemechanismus aufgenommen ist, eine Anzeige-Hülse, die zumindest teilweise in das distale Gehäuseteil einsetzbar ist und in diesem drehbar ist, und eine in das Gehäuseteil einsteckbare Einsteck-Hülse, wobei
   ▪ die Einsteckhülse einen an deren Außenfläche erhabenen Flächenabschnitt aufweist, der zumindest bereichsweise durchsichtig oder transparent gebildet ist, und
   ▪ das Gehäuseteil eine an seinem distalen Rand offene Ausnehmung zur Aufnahme des erhabenen Flächenabschnitts von der offenen Seite der Ausnehmung her aufweist, wobei die Einsteck-Hülse im eingesetzten Zustand drehfest von dem Gehäuseteil aufgenommen ist,
      dadurch gekennzeichnet, dass
   ▪ die Anzeigehülse mit einem Außengewinde versehen ist und die Eisteckhülse auf ihrer Innenseite mit einer Führungsvorrichtung versehen ist, die mit dem Außengewinde der Anzeigehülse zusammenwirkt, um die Anzeigehülse in Abhängigkeit der Drehstellung der anzeigehülse axial zu verstellen.

In einer Ausführungsform der Erfindung haben die einander gegenüber liegenden Innenkanten der in axialer Richtung des distalen Gehäuseteils verlaufenden Ränder der Ausnehmung einen Abstand voneinander, bei dem die Ausnehmungsränder und die jeweiligen Seitenkanten des erhabenen Flächenabschnitts eine Spiel-, Übergangs- oder Press-Passung bilden.

In einer weiteren Ausführungsform der Erfindung verlaufen die Konturlinien der Stoßkante des erhabenen Flächenabschnitts und entsprechend der Stoßkante der Ausnehmung geradlinig und in Umfangsrichtung.

Vorzugsweise können die Konturlinien der Stoßkante des erhabenen Flächenabschnitts und entsprechend der Stoßkante der Ausnehmung aus zwei winklig zueinander verlaufenden Linienabschnitten, die insbesondere spitz zulaufen, gebildet sein, oder
es sind die Konturlinien der Längskanten und der Stoßkante des erhabenen Flächenabschnitts und entsprechend der Längskanten und der Stoßkante der Ausnehmung als Kurvenlinien gebildet, so dass die Umrisse des erhabenen Flächenabschnitts und der Ausnehmung zumindest teilweise oval gestaltet sind, oder die Konturlinien der Längskanten des erhabenen Flächenabschnitts und entsprechend der Längskanten der Ausnehmung verlaufen geradlinig und insbesondere parallel zueinander und die Konturlinien der Stoßkante des erhabenen Flächenabschnitts sowie entsprechend der Stoßkante der Ausnehmung sind als Kurvenlinien gebildet.

In einer weiteren Ausführungsform der erfindungsgemäßen Injektionsvorrichtung weist die Oberseite des erhabenen Flächenabschnitts einen auf diesem gebildeten weiteren Vorsprung auf, insbesondere zur Erreichung eines Lupeneffekt im transparenten Teil des erhabenen Flächenabschnitts.

In noch einer weiteren Ausführungsform der erfindungsgemäßen Injektionsvorrichtung ist eine Verrastungsvorrichtung zur Verrastung von Einsteck-Hülse mit Gehäuseteil in axialer Richtung vorgesehen, wenn die Einsteck-Hülse in ihre Endstellung in das Gehäuseteil eingeschoben ist, wobei die Verrastungsvorrichtung vorzugsweise eine an der Innenfläche des Gehäuseteils angeordnete Aufnahmevorrichtung und einen an der Einsteckhülse angeordneten nach außen abstehendes und mit der Aufnahmevorrichtung zusammenwirkendes Hülsen-Einrastteil aufweist.

Weiterhin bevorzugt weist das Hülsen-Einrastteil eine in einer Ausnehmung des Gehäuseteils verlaufende Zunge auf, die an ihrem proximalen Ende mit der Einsteckhülse verbunden ist und an ihrem distalen Ende ein freies Ende mit einem Rastvorsprung bildet.

In der erfindungsgemäßen Injektionsvorrichtung kann die Aufnahmevorrichtung als Vertiefung auf der Innenfläche des Gehäuseteils oder als Ausnehmung gestaltet sein, wobei die Aufnahmevorrichtung vorzugsweise in Umfangsrichtung eine vorbestimmte Breite hat oder als an der Innenumfangsfläche des Gehäuseteils umlaufende Vertiefung ausgebildet ist.

In der erfindungsgemäßen Injektionsvorrichtung kann das freie Ende als einfach oder mehrfach geteiltes freies Ende mit jeweils einem Rastvorsprung ausgebildet sein und die Aufnahmevorrichtung eine oder mehrere Vertiefungen oder Ausnehmungen aufweisen.

In der erfindungsgemäßen Injektionsvorrichtung können in Umfangsrichtung an der Einsteckhülse mehrere Zungen und mehrere entsprechende, an dem Gehäuseteil angeordnete Aufnahmevorrichtungen vorgesehen sein.

In der erfindungsgemäßen Injektionsvorrichtung können alternativ Paare von Zunge und Aufnahmevorrichtung auf gleicher axialer Lage oder in axialer Richtung zueinander versetzt angeordnet sein.

Ferner kann in der erfindungsgemäßen Injektionsvorrichtung das Gehäuseteil entlang zumindest eines Abschnitts des Randes der Ausnehmung mit einer Dichtung versehen sein. In noch einer weiteren Ausführungsform der erfindungsgemäßen Injektionsvorrichtung sind eine oder mehrere Dichtungen am distalen Ende und/oder an der Innenfläche der Einsteckhülse angeordnet.

Weiterhin können in der erfindungsgemäßen Injektionsvorrichtung mehrere erhabene Flächenabschnitte und entsprechend mehrere Ausnehmungen an der Einsteckhülse vorgesehen sein.

Der erhabene Flächenabschnitt kann mit einer oder mehreren Markierungen versehen sein, die geeignet sind, die Ablesbarkeit der an der Anzeigehülse angegebenen und mit dieser eingestellten Dosis zu erleichtern, wobei die Markierungen auf physikalische oder chemische Weise, insbesondere durch Bedrucken oder Bekleben, aufgebracht sein können.

In einer weiteren Ausführungsform der erfindungsgemäßen Injektionsvorrichtung sind die Längskanten und/oder die Stoßkanten von Ausnehmung und erhabenen Flächenabschnitt derart gestaltet, dass diese formschlüssig ineinander greifen.

Ein weiterer Erfindungsgegenstand ist eine Einsteckhülse für die erfindungsgemäße Injektionsvorrichtung, wobei die Einsteckhülse an ihrer Außenfläche einen erhabenen Flächenabschnitt zu einer drehfesten Aufnahme der Einsteckhülse in einer Ausnehmung eines Gehäuseteils aufweist, wobei der Flächenabschnitt zumindest bereichsweise durchsichtig oder transparent gebildet ist. Weitere besondere Ausführungsformen der erfindungsgemäßen Einsteckhülse sind den oben stehenden Ausführungen im Zusammenhang mit der Beschreibung der erfindungsgemäßen Injektionsvorrichtung zu entnehmen.

In einer weiteren Ausführungsform der erfindungsgemäßen Einsteckhülse sind die Seitenkanten des erhabenen Flächenabschnitts derart gestaltet, dass diese mit den Innenkanten der in axialer Richtung des distalen Gehäuseteils verlaufenden Ränder der Ausnehmung des Gehäuseteils eine Spiel-, Übergangs- oder Press-Passung bilden, wobei vorzugsweise die Konturlinie der Stoßkante des erhabenen Flächenabschnitts geradlinig und in Umfangsrichtung verläuft oder die Konturlinie der Stoßkante des erhabenen Flächenabschnitts aus zwei winklig zueinander verlaufenden Linienabschnitten, die insbesondere spitz zulaufen, gebildet ist, oder die Konturlinien der Längskanten und der Stoßkante des Einstellvorsprungs als Kurvenlinien ausgebildet sind, so dass die Umrisse des erhabenen Flächenabschnitts zumindest teilweise oval gestaltet sind, oder die Konturlinien der Längskanten des erhabenen Flächenabschnitts geradlinig und insbesondere parallel zueinander verlaufen und die Konturlinie der Stoßkante des erhabenen Flächenabschnitts als Kurvenlinie gebildet ist.

In noch einer weiteren Ausführungsform der erfindungsgemäßen Einsteckhülse weist die Oberseite des erhabenen Flächenabschnitts einen auf diesem gebildeten weiteren Vorsprung auf, insbesondere um einen Lupeneffekt im transparenten Teil des weiteren Vorsprungs vorzusehen.

In einer weiteren Ausführungsform weist die erfindungsgemäße Einsteckhülse einen Teil einer Verrastungsvorrichtung zur Verrastung mit einer Aufnahmevorrichtung am Gehäuseteil in der Endstellung der Einsteckhülse auf, wobei vorzugsweise der an der Einsteckhülse angeordnete Teil der Verrastungsvorrichtung ein nach außen abstehendes und mit der Aufnahmevorrichtung zusammen wirkendes Hülsen-Einrastteil aufweist und weiterhin bevorzugt das Hülsen-Einrastteil eine in einer Ausnehmung des Gehäuseteils verlaufende Zunge aufweist, die an ihrem proximalen Ende mit der Einsteckhülse verbunden ist und an ihrem distalen Ende ein freies Ende mit einem Rastvorsprung bildet.

Hierbei kann das freie Ende als einfach oder mehrfach geteiltes freies Ende mit jeweils einem Rastvorsprung ausgebildet sein. Ferner können in Umfangsrichtung an der Einsteckhülse mehrere Zungen vorgesehen sein, wobei diese auf gleicher axialer Lage oder in axialer Richtung zueinander versetzt angeordnet sein können.

Es können auch mehrere erhabene Flächenabschnitte an der erfindungsgemäßen Einsteckhülse vorgesehen sein.

In einer weiteren Ausführungsform der erfindungsgemäßen Einsteckhülse ist der erhabene Flächenabschnitt mit einer oder mehreren Markierungen versehen, die geeignet sind, die Ablesbarkeit der Dosis zu erleichtern, die an einer an einer die Einsteckhülse durchragenden Anzeigehülse angegeben ist und mit dieser eingestellt wird, wobei die Markierungen auf physikalische oder chemische Weise, insbesondere durch Bedrucken oder Bekleben, aufgebracht sind.

In noch einer weiteren Ausführungsform der erfindungsgemäßen Einsteckhülse sind die Längskanten und/oder die Stoßkanten des erhabenen Flächenabschnitts derart gestaltet, dass diese formschlüssig mit Längskanten und/oder Stoßkanten der Ausnehmung des Gehäuseteils ineinander greifen.

In noch einer weiteren Ausführungsform der erfindungsgemäßen Einsteckhülse sind eine oder mehrere Dichtungen am distalen Ende und/oder an der Innenfläche der Einsteckhülse angeordnet.

Noch ein weiterer Erfindungsgegenstand ist ein Dosiseinstell-Mechanismus für eine Injektionsvorrichtung zur Abgabe eines Medikaments, enthaltend eine erfindungsgemäße Einsteckhülse.

Schließlich ist noch ein weiterer Erfindungsgegenstand ein Verfahren zum Zusammenbau einer Injektionsvorrichtung, wobei eine erfindungsgemäße Einsteckhülse in ein Gehäuseteil mit einer am distalen Rand offenen Ausnehmung eingeführt wird.

Im Folgenden wird die Erfindung an Hand der Figuren beschrieben, es zeigen:
- die Figur 1 eine perspektivische Darstellung einer ersten Ausführungsform der erfindungsgemäßen Injektionsvorrichtung, auf deren vorderem Teil eine Abdeckkappe aufgesetzt ist,
- die Figur 2 eine perspektivische schematische Darstellung der ersten Ausführungsform der erfindungsgemäßen Injektionsvorrichtung nach der Figur 1,
- die Figur 3 eine perspektivische schematische Darstellung einer zweiten Ausführungsform der erfindungsgemäßen Injektionsvorrichtung,
- die Figur 4 eine perspektivische schematische Darstellung einer dritten Ausführungsform der erfindungsgemäßen Injektionsvorrichtung,
- die Figur 5 eine perspektivische schematische Darstellung einer vierten Ausführungsform der erfindungsgemäßen Injektionsvorrichtung,
- die Figur 6 eine perspektivische schematische Darstellung einer fünften Ausführungsform der erfindungsgemäßen Injektionsvorrichtung,
- die Figur 7 eine perspektivische schematische Darstellung einer sechsten Ausführungsform der erfindungsgemäßen Injektionsvorrichtung,
- Figur 8 eine Darstellung der Ausführungsform der Verläufe der Kantenlinien einer Ausnehmung und eines erhabenen Flächenabschnitts der erfindungsgemäßen Injektionsvorrichtung im Querschnitt gesehen, wobei die zylindrische Form der Bestandteile der Injektionsvorrichtung und insbesondere des Gehäuses und der Anzeigehülse nicht berücksichtigt sind,
- Figur 9 eine gemäß der Darstellung der Figur 8 gemachte Darstellung einer weiteren Ausführungsform der Verläufe der Kantenlinien der Ausnehmung und des erhabenen Flächenabschnitts der erfindungsgemäßen Injektionsvorrichtung im Querschnitt gesehen,
- Figur 10 eine gemäß der Darstellung der Figur 8 gemachte Darstellung einer weiteren Ausführungsform der Verläufe der Kantenlinien der Ausnehmung und des erhabenen Flächenabschnitts der erfindungsgemäßen Injektionsvorrichtung im Querschnitt gesehen,
- Figuren 11 bis 17 verschiedene Ausführungsformen einer Verrastungsvorrichtung zur Verrastung von Einsteck-Hülse mit Gehäuseteil in axialer Richtung.

Zur Beschreibung der Erfindung werden im Folgenden einige der in der Beschreibung und in den Ansprüchen verwendeten Begriffe erläutert:
Zur Bezeichnung der relativen Positionen von Bestandteilen der erfindungsgemäßen Injektionsvorrichtung werden die Bezeichnungen "proximal" und "distal" verwendet. Dabei bedeutet "proximal" die bei der bestimmungsgemäßen Anwendung der Injektionsvorrichtung zum Patienten hin gerichtete Seite des jeweiligen Bestandteils, also die jeweils zur Injektionsnadel hin gerichtete Seite. Demgemäß bedeutet "distal" die entgegengesetzt zur jeweils "proximalen" Seite liegende Seite des jeweiligen Bestandteils.

Der nachfolgend genannte Behälter zur Aufnahme eines Injektionsmittels kann insbesondere eine Kartusche oder eine Ampulle sein, wobei der Behälter in verschiedenen Ausführungsformen, z.B. zylinderförmig oder mit anderen Querschnittsformen versehen, gestaltet sein kann.

Die Erfindung basiert auf Injektionsvorrichtungen zur Aufnahme und Injektion von medizinischen Injektionsmitteln (Lösung, Suspension, Emulsion), die zur manuellen oder automatischen, mechanischen oder elektromechanischen Verabreichung geeignet sind und die auf dem Prinzip der Verdrängung des Injektionsmittels aus einer Kartusche in eine Injektionsnadel mittels eines in der Kartusche axial geführten Kolbens beruhen. Solche Injektionsvorrichtungen sind entweder als Einweg-Gerät oder als wieder verwendbares Gerät konzipiert.

Die erfindungsgemäße Injektionsvorrichtung weist auf:
- ein Kartuschen-Modul vorzugsweise mit einem Kartuschen-Halter, mit einer Kartusche zur Aufnahme des Injektionsmittels und einer gegebenenfalls daran anbringbaren Injektionsnadel, die an einem Ende der Kartusche oder des Kartuschenhalters angebracht ist und durch die das Injektionsmittel injiziert werden kann,
- einen Abgabemechanismus oder Antriebsmechanismus oder KraftübertragungsMechanismus, durch dessen Betätigung die Abgabe des Injektionsmittels erreicht wird, und
- einen Dosis-Einstellmechanismus zur Einstellung der Dosis, mit der die Menge des Injektionsmittels definiert wird, die bei der Betätigung des Abgabemechanismus abgegeben wird.

Die erfindungsgemäße Injektionsvorrichtung 1 zur Abgabe eines Medikaments weist in einem Ausführungsbeispiel ein Gehäuse zum Einsetzen eines Behälters zur Aufnahme eines Injektionsmittels auf. Das Gehäuse kann aus einem vorderen oder proximalen Gehäuseteil und einem hinteren oder distalen Gehäuseteil gebildet sein. Alternativ kann die erfindungsgemäße Injektionsvorrichtung aus einem einteiligen oder einstückig hergestellten oder aus einem andersartig gestalteten Gehäuse gebildet sein.

Die Kartusche oder der Behälter zur Aufnahme eines Injektionsmittels weist z.B. auf: ein proximales Ende und ein distales Ende, eine zylindrisch oder in anderer Weise verlaufende Längswand mit einer Innenseite und einer Außenseite, eine vordere Öffnung zum Auslassen des Injektionsmittels und eine hintere Öffnung, in die ein entlang der Innenseite bewegbarer Kolben einführbar ist. Der Kolben ist in seiner Ausgangsstellung, d.h. bei vollständig gefülltem Behälter, vorzugsweise am distalen Ende des Behälters gelegen. Der Behälter kann direkt in das Gehäuse mit einer Öffnung an seinem proximalen Ende bzw. gegebenenfalls in ein vorderes Gehäuseteil zum Einsetzen in das Gehäuse eingesetzt sein.

Sofern die Injektionsvorrichtung als wieder verwendbares Gerät ausgelegt ist, wird der Behälter bestimmungsgemäß nach seiner Entleerung gewechselt.

Somit kann der Behälter zur Aufnahme eines Injektionsmittels in eine Behälter-Aufnahmevorrichtung oder ein Behälter-Gehäuse oder einen Behälter-Halter mit einem proximalen Ende und einem distalen Ende eingesetzt sein. Jedoch kann alternativ der Behälter-Halter auch als äußeres Gehäuse dienen. In diesem Falle dient das vordere Gehäuseteil als Teil des äußeren Gehäuses zur Aufnahme des Behälters. Auf das proximale Ende des Behälters kann eine Injektionsnadel (nicht dargestellt) aufsetzbar oder fest aufgesetzt sein, die sich gegebenenfalls durch eine Öffnung am proximalen Ende des äußeren Gehäuses oder des Behälter-Gehäuses und/oder durch eine Öffnung am proximalen Ende des Gehäuses hindurch in den Behälter erstreckt.

Die erfindungsgemäße Injektionsvorrichtung zur Abgabe eines Medikaments weist also insbesondere auf: ein Gehäuse zum Einsetzen des Behälters zur Aufnahme eines Injektionsmittels sowie den Behälter mit einem vorderen, proximalen Ende, das mit einer vorderen zum Auslassen des Injektionsmittels vorgesehenen Öffnung versehen ist, zum Einsetzen in ein proximales Ende des Gehäuses und mit einem hinteren, mit einer hinteren Öffnung versehenen Ende, an der in seiner Ausgangsstellung ein in der axialen Richtung der Kartusche mittels der Abgabevorrichtung verschiebbarer Kolben eingesetzt ist.

Der Dosis-Einstellmechanismus ist gebildet aus:
- einem Gehäuseteil 11, das bei einer zweiteiligen Ausbildung des Gehäuses vorzugsweise das distale Gehäuseteil ist, in das zumindest ein Teil des Abgabemechanismus aufgenommen ist,
- einer Anzeige-Hülse 13, die zumindest teilweise in das distale Gehäuseteil einsetzbar ist und in diesem drehbar ist, und
- einer in das distale Gehäuseteil einsteckbare Einsteck-Hülse 15, wobei die Einsteck-Hülse im eingesetzten Zustand drehfest, vorzugsweise drehfest und in axialer Richtung fixiert, von dem Gehäuse 10 aufgenommen ist.

Dazu sind der Innen-Durchmesser der Einsteck-Hülse 15 und der Außen-Durchmesser der Anzeige-Hülse 13 derart vorgesehen, dass die Anzeige-Hülse 13 in die Einsteck- Hülse 15 einführbar ist.

Der Abgabemechanismus ist gekoppelt mit dem Dosis-Einstellmechanismus. Der Dosis-Einstellmechanismus wirkt mechanisch mit dem Abgabemechanismus zusammen. Dabei kann der Dosis-Einstellmechanismus integraler Bestandteil des Abgabemechanismus oder in Bezug auf den Abgabemechanismus ein getrenntes Bauteil sein.

Der Abgabemechanismus bewirkt die Abgabe des Injektionsmittels, wobei die Dosis des abgegebenen Injektionsmittels mittels des Dosis-Einstellmechanismus eingestellt wird. Zur Abgabe oder Applizierung des Injektionsmittels kann der Abgabemechanismus ein Betätigungselement 17 beispielsweise in Form eines Injektionsknopfes und einen mittels einer Kolbenstange mit dem Injektionsknopf direkt oder indirekt gekoppelten Kolben aufweisen.

Der Dosis-Einstellmechanismus hat also eine Dosis-Einstellfunktion, wobei die DosisEinstellung insbesondere durch eine Einstellung der axialen Position der Anzeige- Hülse 13 gegenüber dem distalen Gehäuseteil bei einer Entkoppelung der Anzeige- Hülse 13 von dem Kolben erfolgen kann.

Die Einsteck-Hülse kann ein Innengewinde aufweisen, in das ein entsprechendes Gewinde-Gegenstück der Anzeigehülse aufnehmbar ist, so dass sich die Anzeigehülse bei Drehung gegenüber der Einsteck-Hülse axial bewegt, um über eine eingestellte Axialposition die abzugebende Dosis an Injektionsmittel vorzugeben.

Gegebenenfalls kann die Einsteckhülse mit weiteren Teilen der Injektionsvorrichtung, z.B. mit dem Kartuschen-Modul, in Verbindung stehen und insbesondere in Eingriff stehen oder mit diesen Teilen verrastet sein.

Die Injektionsvorrichtung hat eine Dosis-Applizierfunktion, durch die die mittels des Dosis-Einstellmechanismus vorgegebene Dosis an Injektionsmittel durch Betätigung einer mit dem Kolben gekuppelten Betätigungsvorrichtung abgegeben wird. Dazu wird die Kolbenstange des Abgabemechanismus beispielsweise axial innerhalb des Gehäuses oder des distalen Gehäuseteils geführt, so dass im Betrieb eine axiale Bewegung der Kolbenstange eine axiale Bewegung des Kolbens in der proximalen Richtung bewirkt, die zur Abgabe des Injektionsmittels führt. Die Betätigungsvorrichtung wird von einem Nutzer der Injektionsvorrichtung und insbesondere einem Patienten oder von medizinischen Fachkräften oder von einer Stellvorrichtung betätigt, um die Abgabe des Injektionsmittels zu bewirken. Die Betätigungs- und Dosiervorrichtung setzt die Betätigungsbewegung des Betätigungselements in eine definierte axiale Bewegung der Kolbenstange um, die wiederum eine definierte Bewegung des Kolbens bewirkt, um mit der Betätigung durch den Patienten eine definierte Menge des Injektionsmittels durch die Injektionsnadel abzugeben.

Die Einsteck-Hülse weist einen in das distale Gehäuseteil einführbaren zylindrischen Grundkörper auf. Zur drehfesten Aufnahme der Einsteck-Hülse 15 durch das distale Gehäuseteil weist die Einsteck-Hülse 15 weiterhin einen an deren Außenfläche abgesetzten oder erhabenen Flächenabschnitt oder Einstellvorsprung 20 auf, der zumindest bereichsweise durchsichtig oder transparent gebildet ist.

In einer weiteren Ausführungsform der Erfindung ist der erhabene Flächenabschnitt 20 mit einer oder mehreren Markierungen (Pfeile, Striche oder ähnliches) versehen, die geeignet sind, durch die Einsteckhülse die Ablesbarkeit der an der Anzeigehülse angegebenen und mit dieser eingestellten Dosis zu erleichtern. Diese Markierungen können z.B. durch Bedrucken oder Bekleben, auf mechanische oder chemische Weise wie Bedrucken, Bekleben, Fräsen, Ätzen oder ähnlichem aufgebracht sein.

Das Gehäuse weist eine an seinem distalen Rand offene Ausnehmung 25 auf, die eine Form hat, dass in diese bei entsprechender Einführung der Einsteck-Hülse 15 in das distale Gehäuseteil 11 in Einschubrichtung E der abgesetzte oder erhabene Flächenabschnitt 20 von dem offenen Rand 26 der Ausnehmung her in die Ausnehmung eingeführt wird.

Die Form des abgesetzten oder erhabenen Flächenabschnitts 20 ist derart vorgesehen, dass der Flächenabschnitt mit vorbestimmter Genauigkeit in Umfangsrichtung in die Ausnehmung eingepasst ist, d.h. die einander gegenüber liegenden Innenkanten der in axialer Richtung des distalen Gehäuseteils verlaufenden Ränder 25a, 25b der Ausnehmung 25 haben einen Abstand voneinander, der ein vorbestimmtes Maß größer oder kleiner als die sich in Umfangsrichtung der Einsteck- Hülse erstreckende Breite B des abgesetzten oder erhabenen Flächenabschnitts 20. Alternativ können die entsprechenden Ausnehmungsränder 25a, 25b und der abgesetzte oder erhabene Flächenabschnitt 20 eine Spiel- Übergangs- oder Press-Passung bilden.

Die Dimensionierung von Ausnehmung und erhabenen Flächenabschnitt 20 als Passung und das Vorsehen der Ausnehmung mit einem offenen Rand 26 ist hinsichtlich der Assemblierung der Injektionsvorrichtung vorteilhaft, insbesondere da diese einfacher und/oder mit besserer Genauigkeit erfolgen kann und somit z. B. ein bessere Automatisierung des Assemblierungsvorganges erfolgen kann.

Die Ausnehmung 25 hat eine Stoßseite oder einen proximalen Rand 26, der einen Anschlag für die axiale Endposition des erhabenen Flächenabschnitts 20 bzw. des verbindenden Rands 25c in der Ausnehmung 25 bildet.

Erfindungsgemäß ist diese auf ihrer Innenseite mit einer Führungsvorrichtung versehen, die mit einem gegebenenfalls vorhandenen Außengewinde der Anzeige-Hülse zusammenwirkt, um die Anzeige-Hülse in Abhängigkeit der Drehstellung der Anzeige-Hülse 13 axial zu verstellen. Durch eine Übergangs- oder Press-Passung kann insbesondere die axiale Stellung der Anzeige- Hülse 13 relativ zum Gehäuseteil 11 in Abhängigkeit der jeweiligen Drehstellung der Anzeige-Hülse 13 genauer vorgesehen werden. Dadurch kann wiederum die Genauigkeit, mit der eine eingestellte Dosis durch die Injektionsvorrichtung abgegeben wird, erhöht werden.

Weiterhin kann durch die Vergrößerung der Dicke des erhabenen Flächenabschnitts 20 z.B. durch Gestaltung desselben als Lupe die Sichtbarkeit der Dosiseinstellung erhöht werden. Dadurch kann ebenfalls die Genauigkeit, mit der eine eingestellte Dosis durch den Benutzer abgegeben wird, verbessert werden.

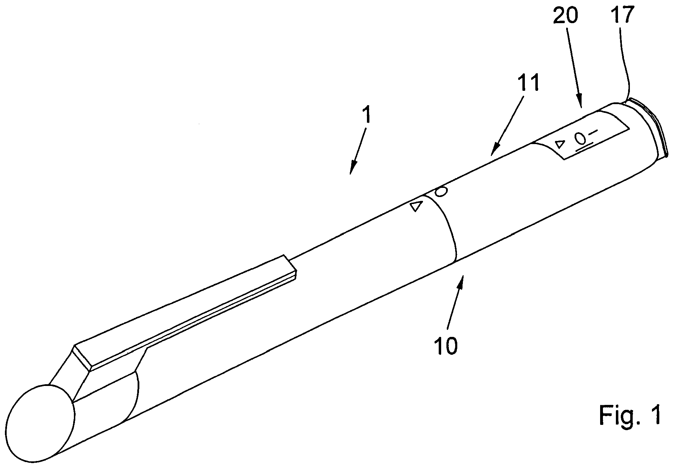

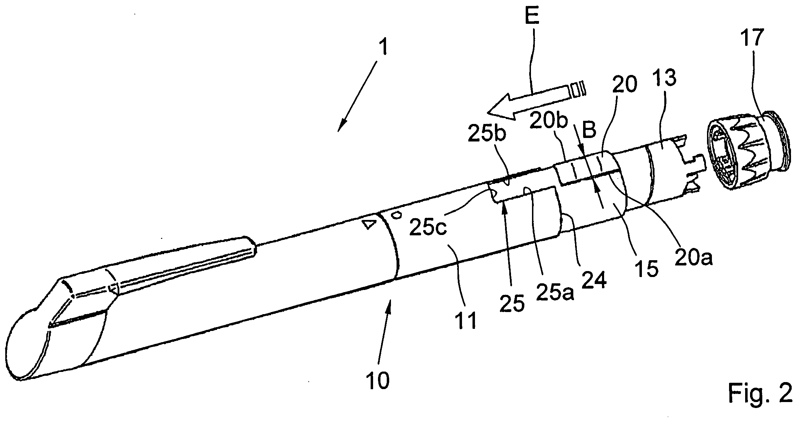

Die Form des abgesetzten oder erhabenen Flächenabschnitts 20 mit seinen in axialer Richtung verlaufenden Seitenkanten 20a, 20b und mit ihrer proximalen Kante oder Stoßkante 20c sowie die Form der Ausnehmung 25 kann verschieden sein:
Wie in den Figuren 1 und 2 gezeigt, kann die Stoßkante 20c des erhabenen Flächenabschnitts und entsprechend die Stoßkante 25c der Ausnehmung geradlinig und in Umfangsrichtung verlaufen.

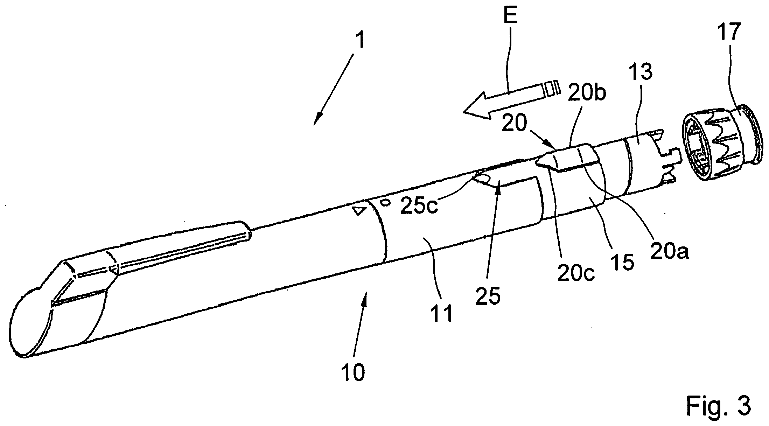

Wie in der Figur 3 gezeigt, kann die Stoßkante 20c des erhabenen Flächenabschnitts 20 und entsprechend die Stoßkante 25c der Ausnehmung aus zwei winklig zueinander verlaufenden Linienabschnitten, die insbesondere spitz zulaufen, gebildet sein.

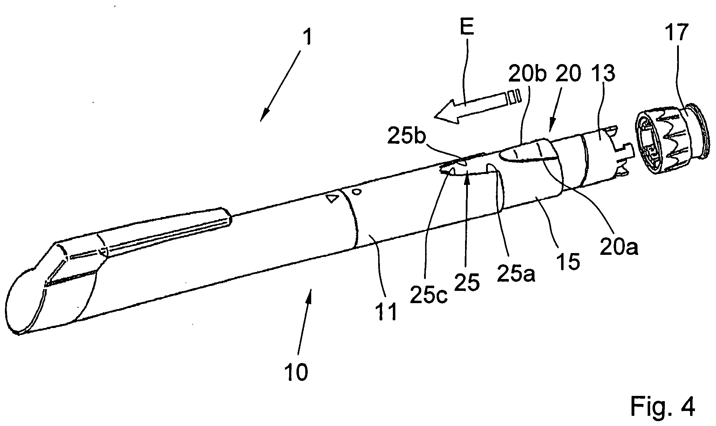

Wie in der Figur 4 gezeigt, können die Längskanten 20a, 20b und die Stoßkante 20c des erhabenen Flächenabschnitts 20 und entsprechend die Längskanten 25a, 25b und die Stoßkante 25c der Ausnehmung als Kurvenlinie gebildet sein, so dass die Umrisse des erhabenen Flächenabschnitts 20 und die Ausnehmung teilweise oval gestaltet sind.

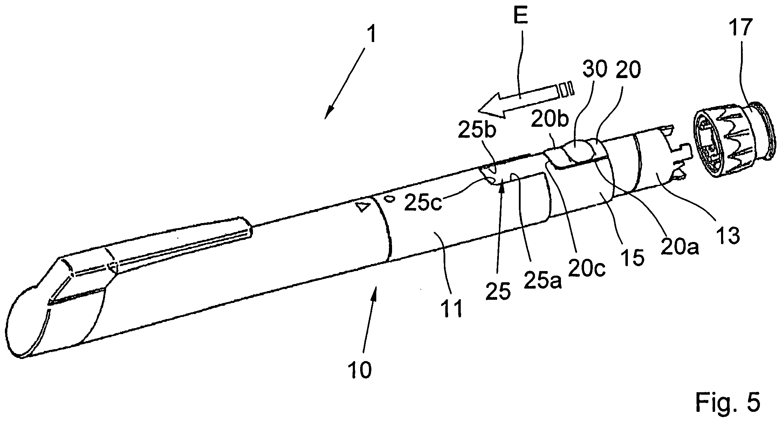

Wie in der Figur 5 gezeigt, kann bei jeder alternativen Gestaltung der Umrisse des erhabenen Flächenabschnitts 20 die Oberseite desselben einen auf diesem gebildeten weiteren Vorsprung 30 aufweisen, insbesondere um einen Lupeneffekt im transparenten Teil des weiteren Vorsprungs 30 vorzusehen.

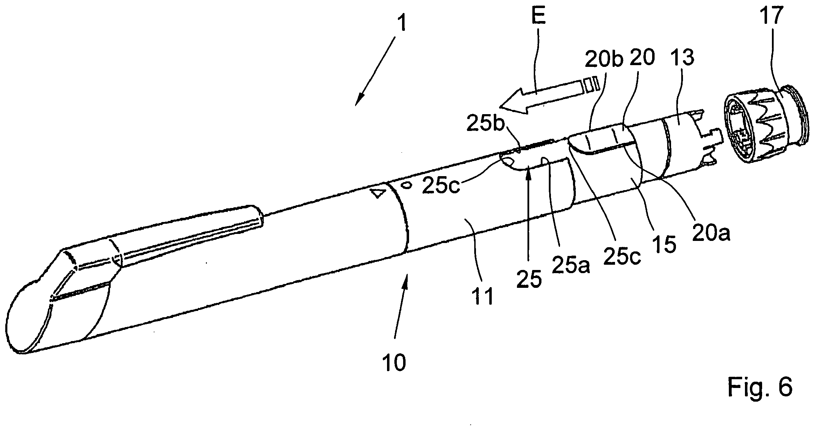

Wie in der Figur 6 gezeigt, können die Längskanten 20a, 20b des erhabenen Flächenabschnitts 20 und entsprechend die Längskanten 25a, 25b der Ausnehmung 25 geradlinig und insbesondere parallel zueinander verlaufen und die Stoßkante 20c des erhabenen Flächenabschnitts 20 sowie entsprechend die Stoßkante 25c der Ausnehmung 25 als Kurvenlinie gebildet sein.

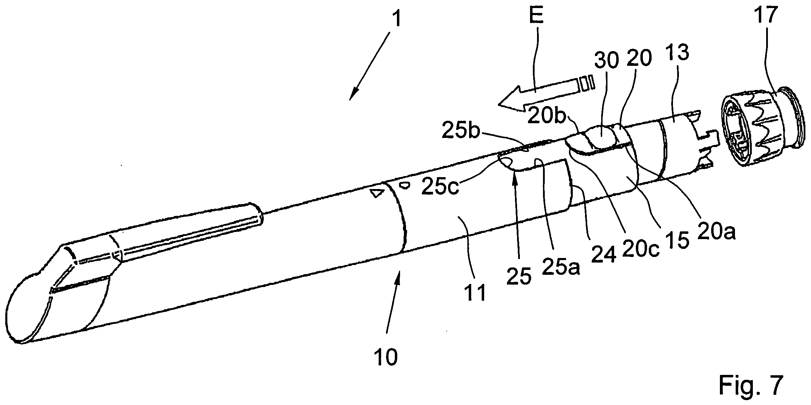

Wie in der Figur 7 gezeigt, kann insbesondere bei der Gestaltung der Umrisse des erhabenen Flächenabschnitts 20 nach der Figur 6 die Oberseite desselben einen auf diesem gebildeten weiteren Vorsprung 30 aufweisen, insbesondere um einen Lupeneffekt im transparenten Teil des weiteren Vorsprungs 30 vorzusehen.

Erfindungsgemäß kann weiterhin eine Verrastungsvorrichtung zur Verrastung von Einsteck-Hülse 15 mit Gehäuseteil 11 in axialer Richtung vorgesehen sein. Dies kann auf verschiedene Weise erfolgen. Z.B. kann an der Außenseite, d.h. der dem Gehäuseteil 11 zugewandten Seite der Einsteck-Hülse 15 ein Rastvorsprung und an einer entsprechenden Stelle der Innenfläche des Gehäuseteils 1 einen Rastnut oder eine Rastvertiefung oder eine Rastausnehmung insbesondere in Form einer Rastöffnung angeordnet sein, die miteinander verrasten, wenn die Einsteck-Hülse 15 in ihre Endstellung in das Gehäuseteil 11 eingeschoben ist.

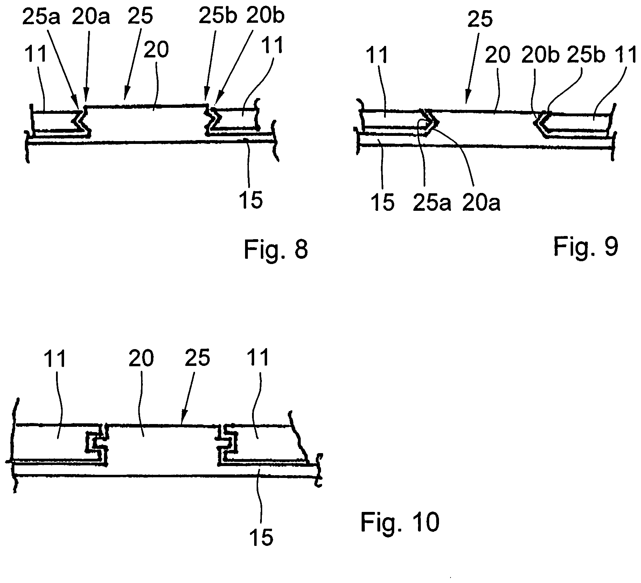

Die Gestaltung der Kanten der Ausnehmung sowie des erhabenen Abschnitts kann in verschiedener Weise vorgesehen sein. Beispielsweise können die Längskanten und/oder die Stoßkanten von Ausnehmung 25 und erhabenem Flächenabschnitt 20 derart gestaltet sein, dass diese formschlüssig ineinander greifen. Dabei können die jeweiligen Kantenflächen von Ausnehmung 25 und erhabenem Flächenabschnitt 20 eine Schwalbenschwanzführung oder eine andersartige Führung bilden. In den Figuren 8 bis 10 sind Gestaltungen der Kantenflächen für die Längskanten des erhabenen Flächenabschnitts und der Ausnehmung dargestellt, die jedoch alternativ oder zusätzlich an den Kantenflächen der Stoßkanten ausgebildet sein können.

In der Figur 8 ist eine Schwalbenschwanzführung dargestellt, bei der die Kantenfläche der Längskante 20a, 20b des erhabenen Flächenabschnitts ein winkliges Führungsteil aufweist, das in eine entsprechende winklige Ausnehmung in der Kantenfläche der Ausnehmung 25 eingreift. In der Figur 9 ist eine Variante der Schwalbenschwanzführung nach der Figur 8 dargestellt, bei der umgekehrt die Kantenfläche der Ausnehmung 25 ein winkliges Führungsteil aufweist, das in eine entsprechende winklige Ausnehmung in der der Längskante 20a, 20b des erhabenen Flächenabschnitts 20 eingreift. Bei der Ausführungsform der Führung nach der Figur 10 ist die Führung in der Kantenfläche der Ausnehmung als Nut gebildet, während in der Kantenfläche des erhabenen Flächenabschnitts ein entsprechendes, in die Nut eingreifendes Gegenstück ausgebildet ist. In analoger Weise kann die Nut auch alternativ in der Kantenfläche der Ausnehmung und das entsprechende Gegenstück in der Kantenfläche des erhabenen Flächenabschnitts ausgebildet sein.

Das distale Gehäuseteil kann entlang des Ausnehmungsrandes mit einer Dichtung versehen sein, um das Innere des distalen Gehäuseteils effizienter gegen Verschmutzung und/oder gegen Feuchtigkeit abzudichten.

Eine Dichtung kann entlang der Kanten der Ausnehmung 25, also entlang der Längskanten 25a, 25b und der Stoßkante, angeordnet sein. Alternativ oder zusätzlich kann eine Dichtung am distalen Ende und an der Innenfläche der Einsteck-Hülse 15 und vorzugsweise entlang des Randes des distalen Endes der Einsteck-Hülse 15 angeordnet sein. Diese Dichtung ist vorzugsweise als am Außenumfang der Einsteck- Hülse 15 umlaufende Dichtung ausgebildet. Dabei kann die Dichtung ringförmig und insbesondere bürstenförmig ausgebildet sein. Bei den vorgenannten Alternativen kann die Dichtung insbesondere rund um die Öffnung des distalen Randes der Einsteck- Hülse 15 verlaufen.

Die vorgenannte Dichtung an der Einsteck-Hülse 15 wirkt zwischen der Innenseite der Einsteck-Hülse 15 und der Außenseite der Anzeige-Hülse 13 und dient insbesondere dazu, den Eintritt von Partikeln oder von Flüssigkeit zwischen die Anzeige-Hülse 13 und die Einsteck-Hülse 15 und somit eine Verschmutzung des Inneren des Gehäuseteils 11 zu verringern oder zu vermeiden.

Alternativ oder zusätzlich kann zur Vermeidung einer Verschmutzung des Inneren des Gehäuseteils 11 eine Dichtung am distalen Ende und an der Außenfläche der Einsteck-Hülse 15 und vorzugsweise entlang des Randes des distalen Endes der Einsteck-Hülse 15 angeordnet sein. Zusätzlich oder alternativ kann eine solche Dichtung auch an der proximalen Seite des erhabenen Flächenabschnitts 20 über den Umfang der Außenseite der Einsteck-Hülse 15 verlaufen.

Es können auch mehrere abgesetzte oder erhabene Flächenabschnitte 20 und entsprechend mehrere Ausnehmungen 25 an der Injektionsvorrichtung 1 vorgesehen sein.

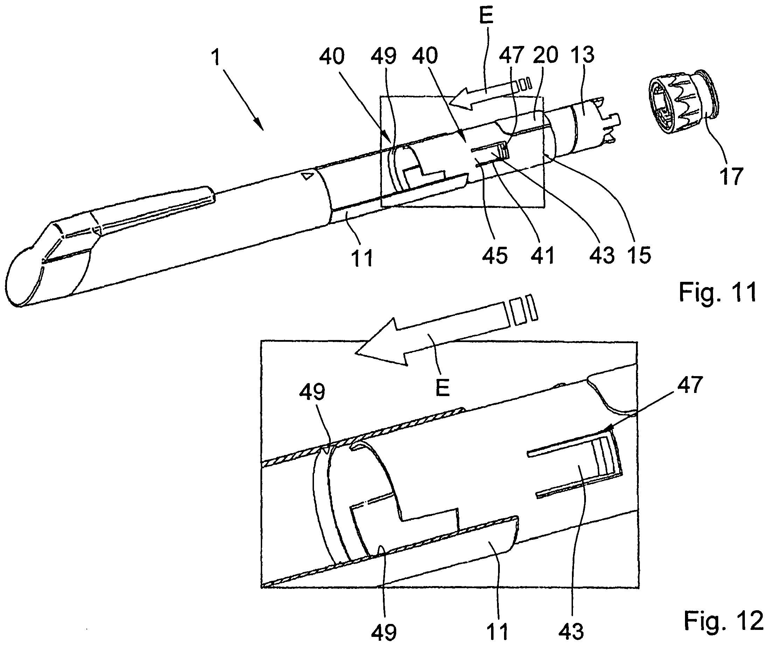

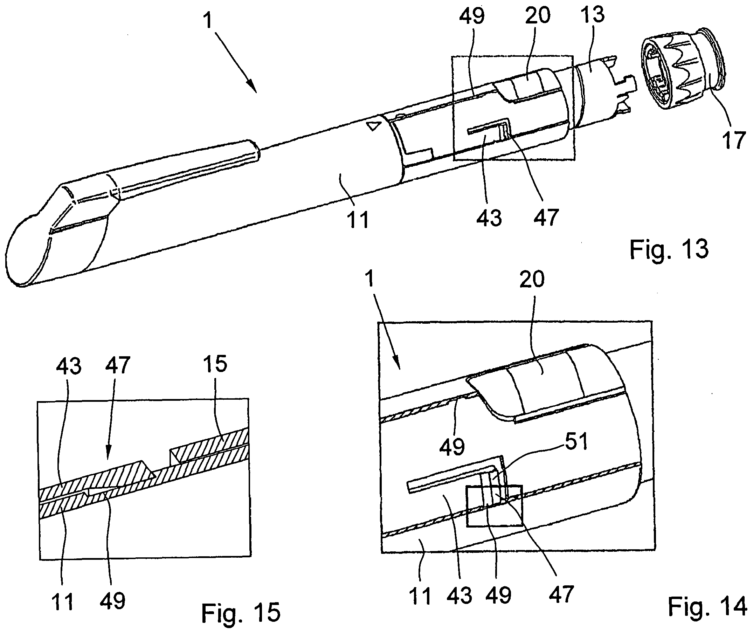

In den Figuren 11 bis 17 sind verschiedene Ausführungsformen der Verrastungsvorrichtung 40 zur Verrastung von Einsteck-Hülse 15 mit Gehäuseteil 11 in axialer Richtung dargestellt.

Die Verrastungsvorrichtung 40 kann eine Aufnahme-Vorrichtung 49 und eine in einer Vertiefung oder einer Ausnehmung 41 des Gehäuseteils 11 verlaufende Lasche oder Zunge 43 aufweisen, die an ihrem proximalen Ende 45 mit der Einsteck-Hülse 15 verbunden ist und an ihrem distalen Ende ein freies Ende 47 bildet. Das freie Ende 47 bildet einen nach außen abstehenden Randbereich 51, 51a, 51b in Form eines Rastvorsprungs aus, der in eine Aufnahme-Vorrichtung 49 eingreift, wenn die Einsteck-Hülse 15 in ihre Endstellung in das Gehäuseteil 11 eingeschoben ist. Der Rastvorsprung 51, 51a, 51b kann durch eine nach außen und winklig verlaufende Erstreckung der Zunge 43 und/oder durch einen am freien Ende 47 ausgebildeten Rastnocken 51, 51a, 51b gebildet sein.

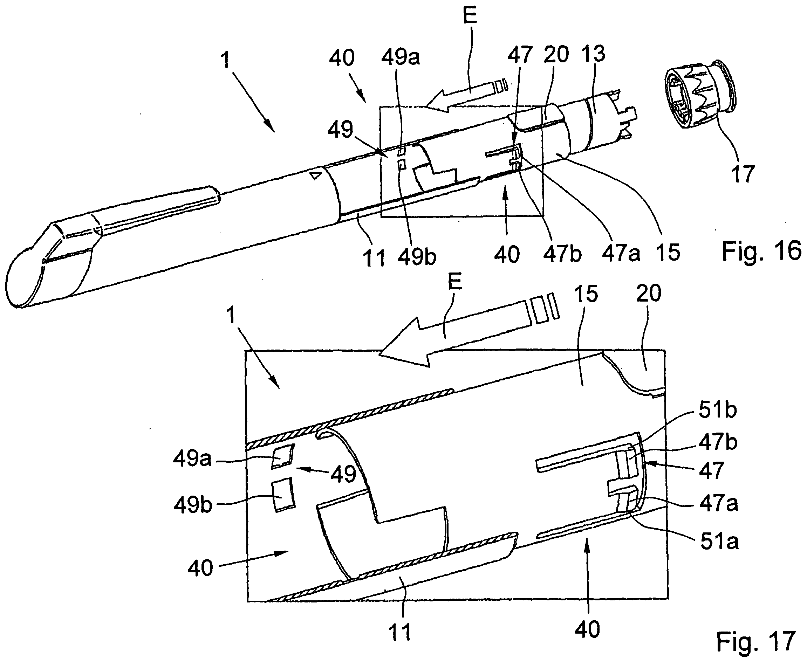

Die mit der Verrastungsvorrichtung 40 zusammen wirkende Aufnahme-Vorrichtung 49 kann, wie es in den Figuren 11 bis 15 dargestellt ist, als Vertiefung auf der Innenfläche des Gehäuseteils 11 gestaltet sein. Alternativ kann die Aufnahme-Vorrichtung 49 als Ausnehmung in Gestalt einer Öffnung (Figuren 16 und 17) gestaltet sein.

In Umfangsrichtung kann sich die Aufnahme-Vorrichtung 49 in einer Breite erstrecken, die mit der Breite des Rastvorsprungs 51, 51a, 51b übereinstimmt und/oder eine Passung ausbildet, mit der die Einsteck-Hülse eine Passung bildet. Eine solche Passung ist vorzugsweise anstelle der vorgenannten mit dem Einstellvorsprung ausgebildeten Passung vorgesehen. Die Aufnahme-Vorrichtung 49 kann sich in Umfangsrichtung über die Breite des Rastvorsprungs 51, 51a, 51b hinaus erstrecken und insbesondere als an der Innenumfangsfläche des Gehäuseteils 11 umlaufende Vertiefung ausgebildet sein (Figuren 11 und 12).

Das freie Ende 47 kann als einfach oder mehrfach geteiltes freies Ende ausgebildet sein, so dass ein oder mehrere Rastvorsprünge 51, 51a, 51b und insbesondere ein oder mehrer Rastnocken vorgesehen sein können. In den Figuren 16 und 17 ist eine Ausführungsform der Verrastungsvorrichtung mit einem einfach geteilten freien Ende und somit mit zwei Rastvorsprüngen 51a, 51b in Form von Rastnocken dargestellt. Dementsprechend weist die Aufnahme-Vorrichtung 49 zwei Vertiefungen oder Ausnehmungen 49a, 49b auf.

Dabei können in Umfangsrichtung auch mehrere Zungen 43 und dementsprechend mehrere Aufnahmevorrichtungen wie Vertiefungen oder Ausnehmungen vorgesehen sein.

Bei den Ausführungsformen mit mehreren Paaren von Zunge 43 und Aufnahmevorrichtung können diese auf gleicher axialer Lage oder in axialer Richtung zueinander versetzt angeordnet sein.

## Patentansprüche

1. Injektionsvorrichtung (1) zur Abgabe eines Medikaments, aufweisend:
- ein Kartuschen-Modul zur Aufnahme einer Kartusche enthaltend ein Injektionsmittel und gegebenenfalls mit einer Aufnahme zum Anbringen einer Nadel, die an einem Ende der Kartusche angebracht ist und durch die das Injektionsmittel injiziert werden kann,
- einen Abgabemechanismus, durch dessen Betätigung die Abgabe des Injektionsmittels erreicht wird,
- einen Dosis-Einstellmechanismus zur Einstellung der Dosis, mit der das Injektionsmittel bei deren Betätigung des Abgabemechanismus abgegeben wird, wobei der Dosis-Einstellmechanismus aufweist: ein Gehäuseteil (11), in das zumindest ein Teil des Abgabemechanismus aufgenommen ist, eine Anzeigehülse (13), die zumindest teilweise in das distale Gehäuseteil (11) einsetzbar ist und in diesem drehbar ist, und eine in das Gehäuseteil (11) einsteckbare Einsteck-Hülse (15),
wobei
- die Einsteckhülse (15) einen an deren Außenfläche erhabenen Flächenabschnitt (20) aufweist, der zumindest bereichsweise durchsichtig oder transparent gebildet ist,
- das Gehäuseteil (11) eine an seinem distalen Rand (24) offene Ausnehmung (25) zur Aufnahme des erhabenen Flächenabschnitts (20) von der offenen Seite der Ausnehmung (25) her aufweist, wobei die Einsteckhülse (15) im eingesetzten Zustand drehfest von dem Gehäuseteil (11) aufgenommen ist, und
die Längskanten (25a, 25b, 20a, 20b) und/oder die Stoßkanten (25c, 20c) von der Ausnehmung (25) und dem erhabenen Flächenabschnitt (20) derart gestaltet ist, dass diese formschlüssig ineinander greifen,
**dadurch gekennzeichnet, dass** die Anzeigehülse mit einem Außengewinde versehen ist und die Einsteckhülse (15) auf ihrer Innenseite mit einer Führungsvorrichtung versehen ist, die mit dem Außengewinde der Anzeigehülse (13) zusammenwirkt, um die Anzeigehülse (13) in Abhängigkeit der Drehstellung der Anzeigehülse (13) axial zu verstellen.

2. Injektionsvorrichtung zur Abgabe eines Medikaments nach dem Anspruch 1, **dadurch gekennzeichnet, dass** die einander gegenüber liegenden Innenkanten der in axialer Richtung des distalen Gehäuseteils verlaufenden Ränder (25a, 25b) der Ausnehmung (25) einen Abstand voneinander haben, bei dem die Ausnehmungsränder (25a, 25b) und die jeweiligen Seitenkanten des erhabenen Flächenabschnitts (20) eine Spiel-, Übergangs- oder Press-Passung bilden.

3. Injektionsvorrichtung zur Abgabe eines Medikaments nach einem oder mehreren der voranstehenden Ansprüche, **dadurch gekennzeichnet, dass** die Konturlinien der Stoßkante (20c) des erhabenen Flächenabschnitts (20) und entsprechend der Stoßkante (25c) der Ausnehmung (25) geradlinig und in Umfangsrichtung verlaufen.

4. Injektionsvorrichtung zur Abgabe eines Medikaments nach einem oder mehreren der voranstehenden Ansprüche 1 bis 2, **dadurch gekennzeichnet, dass** die Konturlinien der Stoßkante (20c) des erhabenen Flächenabschnitts (20) und entsprechend der Stoßkante (25c) der Ausnehmung (25) aus zwei winklig zueinander verlaufenden Linienabschnitten, die insbesondere spitz zulaufen, gebildet sind.

5. Injektionsvorrichtung zur Abgabe eines Medikaments nach einem oder mehreren der voranstehenden Ansprüche 1 bis 2, **dadurch gekennzeichnet, dass** die Konturlinien der Längskanten (20a, 20b) und der Stoßkante (20c) des erhabenen Flächenabschnitts (20) und entsprechend der Längskanten (25a, 25b) und der Stoßkante (25c) der Ausnehmung (25) als Kurvenlinien gebildet sind, so dass die Umrisse des erhabenen Flächenabschnitts (20) und der Ausnehmung (25) zumindest teilweise oval gestaltet sind.

6. Injektionsvorrichtung zur Abgabe eines Medikaments nach einem oder mehreren der voranstehenden Ansprüche 1 bis 2, **dadurch gekennzeichnet, dass** die Konturlinien der Längskanten (20a, 20b) des erhabenen Flächenabschnitts (20) und entsprechend der Längskanten (25a, 25b) der Ausnehmung (25) geradlinig und insbesondere parallel zueinander verlaufen und die Konturlinien der Stoßkante (20c) des erhabenen Flächenabschnitts (20) sowie entsprechend der Stoßkante (25c) der Ausnehmung (25) als Kurvenlinien gebildet sind.

7. Injektionsvorrichtung zur Abgabe eines Medikaments nach einem oder mehreren der voranstehenden Ansprüche 1 bis 6, **dadurch gekennzeichnet, dass** die Oberseite des erhabenen Flächenabschnitts (20) einen auf diesem gebildeten weiteren Vorsprung (30) aufweist, insbesondere um zur Erreichung eines Lupeneffekts im transparenten Teil des erhabenen Flächenabschnitts (20).

8. Injektionsvorrichtung zur Abgabe eines Medikaments nach einem oder mehreren der voranstehenden Ansprüche, **dadurch gekennzeichnet, dass** eine Verrastungsvorrichtung zur Verrastung von Einsteck-Hülse (15) mit Gehäuseteil (11) in axialer Richtung vorgesehen ist, wenn die Einsteck-Hülse (15) in ihre Endstellung in das Gehäuseteil (11) eingeschoben ist.

9. Injektionsvorrichtung zur Abgabe eines Medikaments nach dem Anspruch 8, **dadurch gekennzeichnet, dass** die Verrastungsvorrichtung (40) eine an der Innenfläche des Gehäuseteils (11) angeordnete Aufnahmevorrichtung und ein an der Einsteckhülse (15) angeordnetes nach außen abstehendes und mit der Aufnahmevorrichtung zusammenwirkendes Hülsen-Einrastteil (51, 51 a, 51 b) aufweist.

10. Injektionsvorrichtung zur Abgabe eines Medikaments nach dem Anspruch 9, **dadurch gekennzeichnet, dass** das Hülsen-Einrastteil (51, 51a, 51 b) eine in einer Ausnehmung (41) des Gehäuseteils (11) verlaufende Zunge (43) aufweist, die an ihrem proximalen Ende (45) mit der Einsteckhülse (15) verbunden ist und an ihrem distalen Ende ein freies Ende (47) mit einem Rastvorsprung bildet.

11. Injektionsvorrichtung zur Abgabe eines Medikaments nach dem Anspruch 9 oder 10, **dadurch gekennzeichnet, dass** die Aufnahmevorrichtung (49) als Vertiefung auf der Innenfläche des Gehäuseteils (11) oder als Ausnehmung gestaltet ist.

12. Injektionsvorrichtung zur Abgabe eines Medikaments nach einem oder mehreren der Ansprüche 9 bis 11, **dadurch gekennzeichnet, dass** die Aufnahmevorrichtung (49) in Umfangsrichtung eine vorbestimmte Breite hat oder als an der Innenumfangsfläche des Gehäuseteils (11) umlaufende Vertiefung ausgebildet ist.

13. Injektionsvorrichtung zur Abgabe eines Medikaments nach einem oder mehreren der Ansprüche 9 bis 12, **dadurch gekennzeichnet, dass** das freie Ende (47) als einfach oder mehrfach geteiltes freies Ende mit jeweils einem Rastvorsprung (51a, 51b) ausgebildet ist und die Aufnahmevorrichtung (49) eine oder mehrere Vertiefungen oder Ausnehmungen (49,49a, 49b) aufweist.

14. Injektionsvorrichtung zur Abgabe eines Medikaments nach einem oder mehreren der Ansprüche 9 bis 13, **dadurch gekennzeichnet, dass** in Umfangsrichtung an der Einsteckhülse (15) mehrere Zungen (43) und mehrere entsprechende, an dem Gehäuseteil (11) angeordnete Aufnahmevorrichtungen (49) vorgesehen sind.

15. Injektionsvorrichtung zur Abgabe eines Medikaments nach einem oder mehreren der voranstehenden Ansprüche, **dadurch gekennzeichnet, dass** das Gehäuseteil(11) entlang zumindest eines Abschnitts des Randes der Ausnehmung (25) mit einer Dichtung versehen ist.

## Claims

1. Injection device (1) for dispensing a medicament, comprising:
- a cartridge module for receiving a cartridge containing an injectable preparation and if appropriate with a seat for attachment of a needle, which is mounted on one end of the cartridge and through which the injectable preparation can be injected,
- a dispensing mechanism which is actuated to dispense the injectable preparation,
- a dose-setting mechanism for setting the dose at which the injectable preparation is dispensed when the dispensing mechanism is actuated, the dose-setting mechanism comprising: a housing part (11) in which at least part of the dispensing mechanism is received, a display sleeve (13), which can be fitted at least partially into the distal housing part (11) and is rotatable therein, and an insertion sleeve (15) that can be inserted into the housing part (11),
wherein
- the insertion sleeve (15) has, on its outer face, a raised surface section (20) that is translucent or transparent at least in some areas,
- the housing part (11) has a recess (25) which is open at its distal edge (24) for receiving the raised surface section (20) from the direction of the open end of the recess (25), wherein the insertion sleeve (15) in the inserted state is received in a rotationally fixed manner by the housing part (11), and
the longitudinal edges (25a, 25b, 20a, 20b) and/or the abutting edges (25c, 20c) of the recess (25) and of the raised surface section (20) are designed such that they engage in each other with a form fit,
**characterized in that** the display sleeve is provided with an outer thread and the insertion sleeve (15) is provided on its inner face with a guide device which cooperates with the outer thread of the display sleeve (13) in order to axially adjust the display sleeve (13) as a function of the rotational position of the display sleeve (13).

2. Injection device for dispensing a medicament according to Claim 1, **characterized in that** the mutually facing inner faces of the edges (25a, 25b) of the recess (25) that extend in the axial direction of the distal housing part are spaced apart from each other by a distance at which the recess edges (25a, 25b) and the respective side edges of the raised surface section (20) form a clearance fit, transition fit or interference fit.

3. Injection device for dispensing a medicament according to either or both of the preceding claims, **characterized in that** the contour lines of the abutting edge (20c) of the raised surface section (20) and, accordingly, of the abutting edge (25c) of the recess (25) extend rectilinearly and in the circumferential direction.

4. Injection device for dispensing a medicament according to either or both of preceding Claims 1 and 2, **characterized in that** the contour lines of the abutting edge (20c) of the raised surface section (20) and, accordingly, of the abutting edge (25c) of the recess (25) are formed by two line sections which extend at an angle to each other, and which in particular taper to a point.

5. Injection device for dispensing a medicament according to either or both of preceding Claims 1 and 2, **characterized in that** the contour lines of the longitudinal edges (20a, 20b) and of the abutting edge (20c) of the raised surface section (20) and, accordingly, of the longitudinal edges (25a, 25b) and of the abutting edge (25c) of the recess (25) are formed as curve lines, such that the contours of the raised surface section (20) and of the recess (25) are at least partially oval in shape.

6. Injection device for dispensing a medicament according to either or both of preceding Claims 1 and 2, **characterized in that** the contour lines of the longitudinal edges (20a, 20b) of the raised surface section (20) and, accordingly, of the longitudinal edges (25a, 25b) of the recess (25) extend rectilinearly and in particular parallel to each other, and the contour lines of the abutting edge (20c) of the raised surface section (20) and accordingly of the abutting edge (25c) of the recess (25) are formed as curve lines.

7. Injection device for dispensing a medicament according to one or more of preceding Claims 1 to 6, **characterized in that** the top of the raised surface section (20) has a further projection (30) formed thereon, in particular for achieving a magnifying glass effect in the transparent part of the raised surface section (20).

8. Injection device for dispensing a medicament according to one or more of the preceding claims, **characterized in that** a locking device is provided for locking the insertion sleeve (15) to the housing part (11) in the axial direction, when the insertion sleeve (15) is pushed to its end position into the housing part (11).

9. Injection device for dispensing a medicament according to Claim 8, **characterized in that** the locking device (40) has a receiving device, arranged on the inner face of the housing part (11), and an outwardly protruding sleeve engagement part (51, 51a, 51b) which is arranged on the insertion sleeve (15) and cooperates with the receiving device.

10. Injection device for dispensing a medicament according to Claim 9, **characterized in that** the sleeve engagement part (51, 51a, 51b) has a tongue (43), which extends in a recess (41) of the housing part (11) and which, at its proximal end (45), is connected to the insertion sleeve (15) and, at its distal end, forms a free end (47) with a locking projection.

11. Injection device for dispensing a medicament according to Claim 9 or 10, **characterized in that** the receiving device (49) is designed as a depression on the inner face of the housing part (11) or as a recess.

12. Injection device for dispensing a medicament according to one or more of Claims 9 to 11, **characterized in that** the receiving device (49) has a predetermined width in the circumferential direction or is designed as a circumferential depression on the inner circumferential surface of the housing part (11).

13. Injection device for dispensing a medicament according to one or more of Claims 9 to 12, **characterized in that** the free end (47) is designed as a singly or multiply divided free end in each case with a locking projection (51a, 51b), and the receiving device (49) has one or more depressions or recesses (49, 49a, 49b) .

14. Injection device for dispensing a medicament according to one or more of Claims 9 to 13, **characterized in that** several tongues (43) are provided in the circumferential direction on the insertion sleeve (15), and several corresponding receiving devices (49) arranged on the housing part (11) are provided.

15. Injection device for dispensing a medicament according to one or more of the preceding claims, **characterized in that** the housing part (11) is provided with a seal along at least part of the edge of the recess (25).

## Revendications

1. Dispositif d'injection (1) pour l'administration d'un médicament, présentant :
- un module de cartouche pour recevoir une cartouche comprenant un milieu d'injection et éventuellement avec un logement pour le montage d'une aiguille qui est montée à une extrémité de la cartouche et par le biais de laquelle le milieu d'injection peut être injecté,
- un mécanisme d'administration dont l'actionnement provoque l'administration du milieu d'injection,
- un mécanisme de réglage de dose pour régler la dose avec laquelle le milieu d'injection est administré lors de son actionnement du mécanisme d'administration, le mécanisme de réglage de dose présentant : une partie de boîtier (11) dans laquelle est reçue au moins une partie du mécanisme d'administration, un manchon indicateur (13) qui peut être inséré au moins en partie dans la partie de boîtier distale (11) et qui peut tourner dans celle-ci, et un manchon d'enfichage (15) pouvant être enfiché dans la partie de boîtier (11),
dans lequel
- le manchon d'enfichage (15) présente une portion de surface (20) rehaussée au niveau de sa surface extérieure, qui est formée au moins en partie sous forme translucide ou transparente,
- la partie de boîtier (11) présente un évidement (25) ouvert au niveau de son bord distal (24) pour recevoir la portion de surface rehaussée (20) à partir du côté ouvert de l'évidement (25), le manchon d'enfichage (15), dans l'état inséré, étant reçu de manière solidaire en rotation par la partie de boîtier (11), et
les arêtes longitudinales (25a, 25b, 20a, 20b) et/ou les arêtes de bout (25c, 20c) de l'évidement (25) et de la portion de surface rehaussée (20) étant configurées de telle sorte qu'elles s'engagent par engagement par correspondance de formes les unes dans les autres,
**caractérisé en ce que** le manchon indicateur est pourvu d'un filetage extérieur et le manchon d'enfichage (15) est pourvu sur son côté intérieur d'un dispositif de guidage qui coopère avec le filetage extérieur du manchon indicateur (13) afin de régler axialement le manchon indicateur (13) en fonction de la position de rotation du manchon indicateur (13).

2. Dispositif d'injection pour l'administration d'un médicament selon la revendication 1, **caractérisé en ce que** les arêtes intérieures opposées les unes aux autres des bords (25a, 25b) de l'évidement (25) s'étendant dans la direction axiale de la partie de boîtier distale présentent un espacement l'une de l'autre, au niveau duquel les bords d'évidement (25a, 25b) et les arêtes latérales respectives de la portion de surface rehaussée (20) forment un ajustement avec jeu, un ajustement de transition ou un ajustement serré.

3. Dispositif d'injection pour l'administration d'un médicament selon l'une quelconque ou plusieurs des revendications précédentes, **caractérisé en ce que** les lignes de contour de l'arête de bout (20c) de la portion de surface rehaussée (20) et, de manière correspondante, de l'arête de bout (25c) de l'évidement (25) s'étendent en ligne droite et dans la direction périphérique.

4. Dispositif d'injection pour l'administration d'un médicament selon l'une quelconque ou plusieurs des revendications précédentes 1 et 2, **caractérisé en ce que** les lignes de contour de l'arête de bout (20c) de la portion de surface rehaussée (20) et, de manière correspondante, de l'arête de bout (25c) de l'évidement (25), sont formées à partir de deux portions linéaires s'étendant de manière angulaire l'une par rapport à l'autre, qui convergent notamment en forme de pointe.

5. Dispositif d'injection pour l'administration d'un médicament selon l'une quelconque ou plusieurs des revendications précédentes 1 et 2, **caractérisé en ce que** les lignes de contour des arêtes longitudinales (20a, 20b) et de l'arête de bout (20c) de la portion de surface rehaussée (20) et, de manière correspondante, des arêtes longitudinales (25a, 25b) et de l'arête de bout (25c) de l'évidement (25), sont formées sous forme de lignes courbes de telle sorte que les pourtours de la portion de surface rehaussée (20) et de l'évidement (25) soient configurés au moins en partie sous forme ovale.

6. Dispositif d'injection pour l'administration d'un médicament selon l'une quelconque ou plusieurs des revendications précédentes 1 et 2, **caractérisé en ce que** les lignes de contour des arêtes longitudinales (20a, 20b) de la portion de surface rehaussée (20) et, de manière correspondante, des arêtes longitudinales (25a, 25b) de l'évidement (25), s'étendent en ligne droite et notamment parallèlement les unes aux autres, et les lignes de contour de l'arête de bout (20c) de la portion de surface rehaussée (20), et, de manière correspondante, de l'arête de bout (25c) de l'évidement (25), sont formées sous forme de lignes courbes.

7. Dispositif d'injection pour l'administration d'un médicament selon l'une quelconque ou plusieurs des revendications précédentes 1 à 6, **caractérisé en ce que** le côté supérieur de la portion de surface rehaussée (20) présente une saillie supplémentaire (30) formée sur celle-ci, en particulier afin d'obtenir un effet de loupe dans la partie transparente de la portion de surface rehaussée (20) .

8. Dispositif d'injection pour l'administration d'un médicament selon l'une quelconque ou plusieurs des revendications précédentes, **caractérisé en ce qu'**il est prévu un dispositif d'encliquetage pour l'encliquetage du manchon d'enfichage (15) avec la partie de boîtier (11) dans la direction axiale, lorsque le manchon d'enfichage (15) est enfoncé dans sa position terminale dans la partie de boîtier (11).

9. Dispositif d'injection pour l'administration d'un médicament selon la revendication 8, **caractérisé en ce que** le dispositif d'encliquetage (40) présente un dispositif de réception disposé au niveau de la surface intérieure de la partie de boîtier (11) et une partie d'encliquetage de manchon (51, 51a, 51b) disposée au niveau du manchon d'enfichage (15), faisant saillie vers l'extérieur et coopérant avec le dispositif de réception.

10. Dispositif d'injection pour l'administration d'un médicament selon la revendication 9, **caractérisé en ce que** la partie d'encliquetage de manchon (51, 51a, 51b) présente une langue (43) s'étendant dans un évidement (41) de la partie de boîtier (11), qui est connectée au niveau de son extrémité proximale (45) au manchon d'enfichage (15) et qui forme au niveau de son extrémité distale une extrémité libre (47) avec une saillie d'encliquetage.

11. Dispositif d'injection pour l'administration d'un médicament selon la revendication 9 ou 10, **caractérisé en ce que** le dispositif de réception (49) est configuré sous forme de renfoncement sur la surface intérieure de la partie de boîtier (11) ou sous forme d'évidement.

12. Dispositif d'injection pour l'administration d'un médicament selon l'une quelconque ou plusieurs des revendications 9 à 11, **caractérisé en ce que** le dispositif de réception (49) présente dans la direction périphérique une largeur prédéterminée ou est réalisé sous forme de renfoncement périphérique au niveau de la surface périphérique intérieure de la partie de boîtier (11).

13. Dispositif d'injection pour l'administration d'un médicament selon l'une quelconque ou plusieurs des revendications 9 à 12, **caractérisé en ce que** l'extrémité libre (47) est réalisée sous forme d'extrémité libre divisée une ou plusieurs fois avec à chaque fois une saillie d'encliquetage (51a, 51b) et le dispositif de réception (49) présente un ou plusieurs renfoncements ou évidements (49, 49a, 49b).

14. Dispositif d'injection pour l'administration d'un médicament selon l'une quelconque ou plusieurs des revendications 9 à 13, **caractérisé en ce que** dans la direction périphérique au niveau du manchon d'enfichage (15) sont prévus plusieurs langues (43) et plusieurs dispositifs de réception (49) correspondants, disposés au niveau de la partie de boîtier (11).

15. Dispositif d'injection pour l'administration d'un médicament selon l'une quelconque ou plusieurs des revendications précédentes, **caractérisé en ce que** la partie de boîtier (11) est pourvue d'un joint d'étanchéité le long d'au moins une portion du bord de l'évidement (25).
